# EUROPEAN PATENT APPLICATION

(11) **EP 4 578 469 A1**
(43) Date of publication of application: **02.07.2025**
(21) Application number: 24221683.6
(22) Date of filing: 19.12.2024
(51) Int. Cl.: A61L 27/30, A61L 27/54, A61L 27/56, A61L 31/08, A61L 31/14, A61L 31/16

(54) **MEDICAL IMPLANT COMPONENTS WITH A BIOCOMPATIBLE-BIOACTIVE COMPOSITE MATERIAL LAYER, METHOD OF MAKING THE SAME AND APPLICATIONS OF THE SAME**

(30) Priority: 26.12.2023 US 202363614853 P
(71) Applicant: Innojet Technology Co., Ltd., Taoyuan City 320017 (TW)
(72) Inventor: CHANG, Jen-Hsien, 320017 Taoyuan City (TW); TANG, Wei-Cheng, 320017 Taoyuan City (TW); HUANG, Yang-Sheng, 320017 Taoyuan City (TW); TSAI, Yu-Yen, 320017 Taoyuan City (TW)
(74) Representative: Kilger, Ute

(57) **Abstract**

The present disclosure relates to medical implant components comprising a biocompatible-bioactive composite material layer (BACL), methods of making the medical implant components and applications of the medical implant components.

## Description

### TECHNICAL FIELD OF THE INVENTION

The present disclosure relates to medical implant components comprising a biocompatible-bioactive composite material layer (BACL), the BACL comprising component (a): a biocompatible metal or alloy, a biocompatible ceramic or any mixtures thereof, and component (b): an organic bioactive ingredient, and a process of making the medical implant components. The present disclosure also relates to methods of making the medical implant components and applications of the medical implant components.

### BACKGROUND OF THE INVENTION

Medical implant components may be used in various clinical and healthcare applications in order to provide benefits for physicians, surgeons, registered professional nurses and patients during or after medical treatments. Thanks to developments in material science and medical sciences, the prognosis of patients requiring surgery or medical implants is continuously improving. For example, artificial joints may improve motor functions of subjects or patients suffering from age-related conditions or diseases and may extend the lifespan of body parts in subjects or patients.

Infection by microorganisms or viruses is a common complication during or after surgery, and requires debridement, extensive antibiotic use, blood transfusions, and may lead to prolonged hospitalization. This places a burden on patients and consumes medical resources. In severe cases, it may lead to sepsis, the need for amputation, or even death. Antibiotics are widely used to reduce the risk of infection during surgery, either by incorporating them into implants or applying them to surfaces. Bacterial growth can be inhibited and the impact of antibiotics on tissue repair can be minimized by controlling the release of the drug. To control the release of the drug and maintain overall mechanical strength of implants, antibiotics carriers (organics such as polylactic acid, polyethylene glycol, and bone cement; inorganics such as hydroxyapatite, calcium phosphate, and other materials) are used to load antibiotics to meet the above requirements. The layer formed by such antibiotic carriers with antibiotics is referred to as an "organic antimicrobial membrane."

To enhance the performance of medical implants, the organic antimicrobial membrane may be applied as a coating on the surface of medical implants. However, existing organic antimicrobial membranes may still exhibit certain defects or disadvantages such as insufficient durability or mechanical strength, or have a high risk of contaminating the medical implants. In addition, costs of existing processes for preparing organic antimicrobial membrane coated medical implants may be high, and applicability of the processes may be limited in view of the materials of the organic antimicrobial membrane or medical implants.

Hence, there is still a need to develop novel and cost-effective medical implants and processes for preparing the same.

### SUMMARY OF THE INVENTION

The present disclosure thus relates to a medical implant component, comprising:
(a) a biocompatible substrate (S), and
(b) a biocompatible-bioactive composite material layer (BACL), having a porosity of 0.5% to 40%, preferably of 30% or less, above or on the substrate;

wherein the biocompatible substrate (S) is made from a material or materials selected from the group consisting of a biocompatible polymeric material, a first biocompatible metal or alloy and a first biocompatible ceramic, and
wherein the BACL is made of a composite material comprising component (1): a second biocompatible metal or alloy, a second biocompatible ceramic or any mixtures thereof, and component (2): an organic bioactive ingredient.

The present disclosure also relates to a process of making a medical implant component described herein, comprising the following steps:
(i) providing a biocompatible substrate (S) in a deposition chamber;
(ii) reducing the pressure in the deposition chamber to less than 2.5 torr, e.g., less than 2.35 torr, less than 2.2 torr; and
(iii) depositing a composite material of biocompatible-bioactive composite material layer (BACL) via aerosol deposition (AD) to form BACL above or on the biocompatible substrate, and
wherein the composite material comprises component (1): a biocompatible metal or alloy, a biocompatible ceramic or any mixtures thereof, and component (2): an organic bioactive ingredient.

In one aspect, the present disclosure provides a medical implant component comprising a biocompatible substrate (S), an assisting dense layer (ADL) on the substrate and a biocompatible-bioactive composite material layer (BACL) on the assisting dense layer (ADL), i.e., the ADL is located between the biocompatible substrate and the BACL, wherein the ADL is made of a biocompatible metal or alloy, a biocompatible ceramic or any mixtures thereof, and
the ADL has a surface roughness of less than 0.3 µm.

In one aspect, the present disclosure provides a process of making a medical implant component described herein, comprising the following steps:
(i) providing a biocompatible substrate (S) in a deposition chamber;
(ii) reducing the pressure in the deposition chamber to less than 2.5 torr, e.g., less than 2.35 torr, less than 2.2 torr; and
(iii) depositing the composite material of the BACL via aerosol deposition (AD) to form the BACL above or on the biocompatible substrate,
wherein the material of the ADL comprises a biocompatible metal or alloy, biocompatible ceramic or any mixtures thereof.

In one aspect, the present disclosure provides a process of making a medical implant component described herein, comprising the following steps:
(i) providing a biocompatible substrate (S) in a deposition chamber;
(ii) reducing the pressure in the deposition chamber to less than 2.5 torr, e.g., less than 2.35 torr, less than 2.2 torr;
(ii') depositing a material of an assisting dense layer (ADL) on the substrate via aerosol deposition (AD), and
(iii') depositing the composite material of the BACL via aerosol deposition (AD) to form the BACL above or on the ADL,
wherein the material of the ADL comprises a biocompatible metal or alloy, biocompatible ceramic or any mixtures thereof.

In one aspect, the present disclosure provides the medical implant component as a part or the entirety of an artificial joint, an insert associated with an artificial joint, a stent, an intervertebral disc, a screw, an artificial bone plate, an interbody spacer or a permanent or temporary anchorage device.

In one embodiment, the BACL has a thickness of 0.1 µm to 80 µm, preferably 0.5 to 50 µm, more preferably 1 to 6 µm, e.g., from 0.75 µm to 15 µm, from 1.5 µm to 25 µm, about 10 µm, about 20 µm, about 30 µm, about 40 µm, or any reasonable numeric range constituted by the values noted above as the terminal point or the single point.

In any preceding embodiments, the medical implant component has a surface roughness of 0.3µm or higher.

In any preceding embodiments, the BACL has a surface roughness of 0.05 µm to 4 µm, e.g., 0.1 µm to 3.5 µm, 0.15 µm to 3 µm, 0.075 µm to 3.75 µm, 0.2 µm to 2.5 µm, etc.

In any preceding embodiments, the BACL is formed from primary particles having D50 ranging from 0.1 µm to 10 µm, preferably ranging from 0.5 µm to 3 µm.

In any preceding embodiments, component (1) in the BACL is selected from the group consisting of oxides of one or more of aluminum, silicon, titanium and zirconium; nitrides of one or more of aluminum, silicon, titanium and zirconium; carbides of one or more of aluminum, silicon, titanium and zirconium; oxide-nitride of one or more of aluminum, silicon, titanium and zirconium; oxide-carbide of one or more of aluminum, silicon, titanium and zirconium; nitride-carbide one or more of aluminum, silicon, titanium and zirconium; oxide-nitride-carbide of one or more of aluminum, silicon, titanium and zirconium; phosphates of calcium; hydroxylapatites; halogenated hydroxylapatites; carbonated hydroxylapatites; halogenated-carbonated hydroxylapatites; and any mixtures of the foregoing.

In any preceding embodiments, component (2) in the BACL is selected from the group consisting of antibiotics, platelet-rich plasma (PRP), collagen, steroids, nucleic acids, antibodies, functional fragments of antibodies; and any mixtures of the foregoing.

In any preceding embodiments, component (2) in the BACL is present in an amount of 0.3 wt.% to 25 wt.%, preferably 0.5 wt.% to 20 wt.%, based on the total weight of the composite material.

In any preceding embodiments, the variation of thickness of the BACL is less than 10%, e.g., less than 8%, less than 5%, etc.

In any preceding embodiments, the ADL, if present, has a thickness of 0.5 µm to 10 µm, preferably 1 µm to 3 µm.

In any preceding embodiments, the adhesion strength between the substrate and BACL is greater than 3B, as measured by ASTM D3359. If the ADL is present, (i) the adhesion between the ADL and the substrate is at least 4B, as measured by ASTM D3359, (ii) the adhesion between the ADL and the BACL is at least 4B as measured by ASTM D3359 or (iii) both (i) and (ii). In any preceding embodiments, the adhesion between the film stack (i.e., ADL+BACL) and substrate is greater than 3B, preferably greater than 4B, as measured by ASTM D3359.

In any preceding embodiments, if the ADL is present, the variation of thickness of the ADL is less than 10%.

In any preceding embodiments, if the ADL is present, the ADL has a porosity of less than 1%, preferably determined under FE-SEM at a magnification ratio of 10000X.

In any preceding embodiments, if the ADL is present, the BACL has a surface roughness of 0.05 µm to 4 µm, e.g., 0.1 µm to 3.5 µm, 0.15 µm to 3 µm, 0.075 µm to 3.75 µm, 0.2 µm to 2.5 µm, etc.

In any preceding embodiments, if the ADL is present, its biocompatible metal or alloy, biocompatible ceramic or any mixtures thereof are different from component (1) of the composite material of the BACL.

In any preceding embodiments, if the ADL is present, its biocompatible metal or alloy, biocompatible ceramic or any mixtures thereof are the same as component (1) of the composite material of the BACL.

In any preceding embodiments, the medical implant component comprises at least two biocompatible-bioactive composite material layers (BACLs).

In any preceding embodiments, the biocompatible polymeric material is selected from the group consisting of polytetrafluoroethene (PTFE), polyetheretherketone (PEEK), polyethylene (PE), polyurethane (PU) and polyvinylchloride (PVC).

In any preceding embodiments, the PE is low-density polyethylene (LDPE), high-density polyethylene (HDPE) or ultra-high molecular weight polyethylene (UHMWPE).

In any preceding embodiments, the biocompatible polymeric material has a hardness of at least 400 HV (Vickers hardness), preferably up to 600 HV, more preferably up to 1000 HV; or at least a Shore D of 50 (Shore hardness), preferably at least a Shore D of 55, more preferably at least a Shore D of 60, most preferably at least a Shore D of 65.

In any preceding embodiments, the biocompatible metal or alloy for the substrate (S) is selected from the group consisting of titanium (Ti) or an alloy thereof, zirconium (Zr) or an alloy thereof, tantalum (Ta) or an alloy thereof, niobium (Nb) or an alloy thereof, stainless steel, cobalt-chromium-molybdenum (Co-Cr-Mo) alloy, and Ti-6Al-4V alloy.

In any preceding embodiments, the biocompatible ceramic for the substrate (S) is selected from the group consisting of oxide, carbide, nitride, or nitro-carbide of any of the following elements: silicon (Si), titanium (Ti), tantalum (Ta), tungsten (W), zirconium (Zr), niobium (Nb), chromium (Cr) and aluminum (Al).

In any preceding embodiments, the AD is carried out with a carrier gas selected from the group consisting of N₂, O₂, Ar, He, clean dry air (CDA) and any combinations thereof.

In any preceding embodiments, the AD is carried out at a flow rate of a carrier gas of 300 to 1500 l/hr.

In any preceding embodiments, the temperature of the substrate during the deposition of the ADL and/or the BACL ranges from 5°C to 50°C, e.g., at least 15°C, preferably not higher than 35°C.

In any preceding embodiments, the substrate (S) is polished to exhibit a surface roughness of at least 0.2 µm.

### BRIEF DESCRIPTION OF THE DRAWINGS

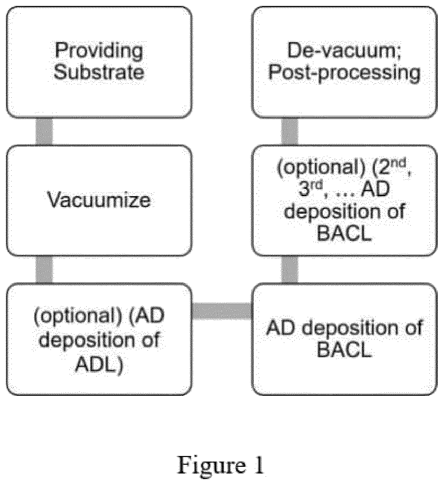

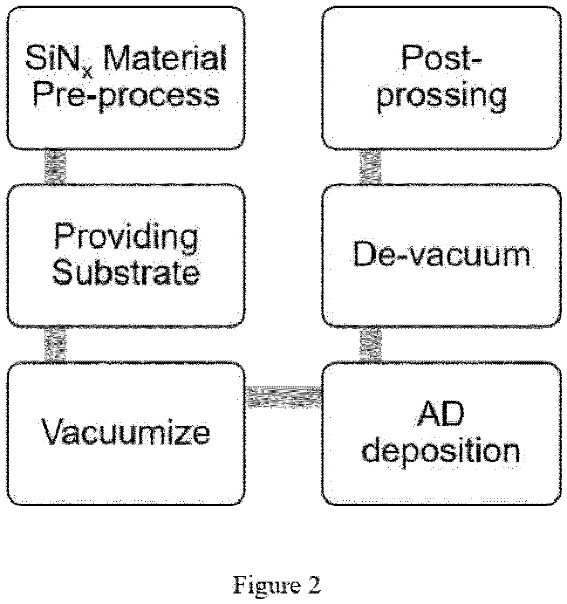

Figures 1 and 2 show illustrative schemes of the process of making the inventive medical implant.

### DETAILED DESCRIPTION OF THE INVENTION

In order to facilitate understanding of the disclosure herein, terms as used herein are hereby defined below.

In the context of the specification and the claims, the singular forms "a," "an" and "the" include plural referents, unless specifically indicated otherwise. Unless otherwise stated, any and all examples or exemplary language (e.g., "such as") provided herein are merely used for better illustration of the present invention, instead of limiting the scope of the present invention.

It is to be understood that any numerical range recited in this specification is intended to include all sub-ranges encompassed therein. For example, a range from "50 to 70°C" includes all sub-ranges and specific values between the stated minimum value of 50°C and the stated maximum value of 70°C, inclusive, e.g., from 58°C to 67°C, and from 53°C to 62°C, 60°C or 68°C. Since the numerical ranges disclosed are continuous, they contain each numerical value between the minimum and maximum value. Unless otherwise specified, the various numerical ranges indicated in this specification are approximate.

In the present invention, the term "about" refers to an acceptable deviation of a given value measured by a person of ordinary skill in the art, depending, in part, on how to measure or determine the value.

In the present disclosure, the term "biocompatible" or "biocompatibility" means having an ability to be in contact with a living system without producing an adverse effect, e.g., (severe) allergic response, damage to cells, tissues or organs in a living body, etc.

In the present disclosure, the term "porosity" refers to the level of pore space in a material.

### Medical implant components

(In)organic antimicrobial membranes may be applied as a coating on the surface of medical implants to avoid infection, improve prognosis and/or provide long-term treatment after surgery. Existing (in)organic antimicrobial membranes include those using organic gelling or polymeric materials such as gelatin, poly(lactic acid) (PLA, including poly-D,L lactic-acid (PDLLA)), poly(glycolic acid) (PGA), etc., or hydroxylapatites, and the antimicrobial substances can be loaded by immersion of the membrane or co-precipitation from a solution. However, the thickness of the (in)organic antimicrobial membranes may be limited due to the process of preparation or the requirement of (mechanical) strength; the thickness is also difficult to control. A possible improvement is to use a 3D transitional ceramic structure, but this may lead to increase costs or complexity of production, or the risk of contaminating the materials.

Hence, in the present disclosure, a medical implant component comprising a biocompatible substrate (S) and a biocompatible-bioactive composite material layer (BACL), having a porosity of 0.5% to 40%, above or on the substrate is provided, wherein the BACL is made of a composite material comprising component (1): a biocompatible metal or alloy, a biocompatible ceramic or any mixtures thereof, and component (2): an organic bioactive ingredient. In one aspect, the medical implant component comprises a biocompatible substrate (S), an assisting dense layer (ADL) on the substrate and a biocompatible-bioactive composite material layer (BACL) on the assisting dense layer (ADL), i.e., the ADL is located between the biocompatible substrate and the BACL, wherein the ADL is made of a biocompatible metal or alloy, a biocompatible ceramic or any mixtures thereof.

Details of the components or layers of the medical implant component are described below.

### Biocompatible substrates

In the present disclosure, biocompatible substrates known in the art may be used once they have the desired characteristics, such as sufficient mechanical strength (hardness, robustness, etc.), chemical inertness, biocompatibility, etc. For example, the materials for making the biocompatible substrates may have a hardness of at least 400 HV (Vickers hardness) or at least a Shore D of 50 (Shore hardness). In various embodiments, the biocompatible substrate can be made from a material or materials selected from the group consisting of a biocompatible polymeric material, a biocompatible metal or alloy and a biocompatible ceramic.

Examples of the biocompatible polymeric material include, but are not limited to, polyketones such as polyetheretherketone (PEEK); (halogenated) polyalkylenes such as polyethylene (PE), poly(ethylene-propylene), polytetrafluoroethene (PTFE) and polyvinylchloride (PVC); polyurethanes, etc. In various embodiments, the PE is low-density polyethylene (LDPE), high-density polyethylene (HDPE) or ultra-high molecular weight polyethylene (UHMWPE).

Examples of the biocompatible metal or alloy include, but are not limited to, titanium (Ti) or an alloy thereof, zirconium (Zr) or an alloy thereof, tantalum (Ta) or an alloy thereof, niobium (Nb) or an alloy thereof, stainless steel, cobalt-chromium-molybdenum (Co-Cr-Mo) alloy, and Ti-6Al-4V alloy.

Examples of the biocompatible ceramic include, but are not limited to, oxide, carbide, nitride, or nitro-carbide of any of the following elements: silicon (Si), titanium (Ti), tantalum (Ta), tungsten (W), zirconium (Zr), niobium (Nb), chromium (Cr) and aluminum (Al).

In one embodiment, the biocompatible substrate has a surface roughness (Ra) of less than 0.3um; or higher than 0.01um. In one embodiment, the biocompatible substrate has a linear coefficient of thermal expansion ranging from 6*10⁻⁶ to 18*10⁻⁵.

### Biocompatible-bioactive composite material layer (BACL)

In the present disclosure, a biocompatible-bioactive composite material layer (BACL) is used to achieve the above-mentioned advantages, e.g., exhibiting high stability of structure, providing prolonged/sustained/long-term release of bioactive ingredient(s), etc.

The BACL is made of a composite material comprising component (1): a biocompatible metal or alloy, a biocompatible ceramic or any mixtures thereof, and component (2): an organic bioactive ingredient. The composite material may be in solid forms such as admixtures, blends, powders, etc., and is substantially homogeneous. In one embodiment, the composite material is a solid dispersion comprising components (1) and (2). In one embodiment, the composite material is a solid dispersion consisting of components (1) and (2).

The material of component (1), i.e., a biocompatible metal or alloy, a biocompatible ceramic or any mixtures thereof, is selected from the group consisting of oxides of one or more of aluminum, silicon, titanium and zirconium; nitrides of one or more of aluminum, silicon, titanium and zirconium; carbides of one or more of aluminum, silicon, titanium and zirconium; oxide-nitride of one or more of aluminum, silicon, titanium and zirconium; oxide-carbide of one or more of aluminum, silicon, titanium and zirconium; nitride-carbide one or more of aluminum, silicon, titanium and zirconium; oxide-nitride-carbide of one or more of aluminum, silicon, titanium and zirconium; phosphates of calcium; hydroxylapatites; halogenated hydroxylapatites; carbonated hydroxylapatites; halogenated-carbonated hydroxylapatites; and any mixtures of the foregoing.

The material of component (2), i.e., an organic bioactive ingredient, is selected from the group consisting of antibiotics, platelet-rich plasma (PRP), collagen, steroids, nucleic acids, antibodies, functional fragments of antibodies; and any mixtures of the foregoing. In one embodiment, component (2) can be antibiotics such as β-lactams, glycopeptides, lipopeptides, rifamycins, macrolides, aminoglycoside, fluoroquinolones, lincosamides (e.g., lincomycim, clindamycin, pirilimycin), tetracyclines, fusidic acid and the like, etc. In one embodiment, component (2) can be substances which are beneficial to or can enhance repair of cells or tissues, such as platelet-rich plasma (PRP), steroids, DNA/RNA, antibodies. In one embodiment, the organic bioactive ingredient is insoluble or hardly soluble in water or aqueous media, such as physiological media (e.g., PBS, saline, etc.).

In one embodiment, the medical implant component comprises a biocompatible substrate (S) and two or more biocompatible-bioactive composite material layers (BACLs). In one embodiment, the medical implant component consists of a biocompatible substrate (S) and a biocompatible-bioactive composite material layer(s) (BACL(s)). In one embodiment, the medical implant component consists of a biocompatible substrate (S) and a biocompatible-bioactive composite material layer(s) (BACL(s)), wherein the BACL(s) consists of hydroxylapatites; halogenated hydroxylapatites; carbonated hydroxylapatites; halogenated-carbonated hydroxylapatites; or any mixtures of the foregoing.

In one embodiment, the biocompatible-bioactive composite material layer(s) (BACL(s)) is located on the substrate or above the substrate (e.g., when an assisting dense layer (ADL), described below, is present). The BACL(s) each independently shall have a porosity of 0.5% to 40%, preferably 0.8% to 35%, e.g., at least 1%, at least 5%, at least 7.5%, at least 10%, up to 37.5%, up to 30%, up to 25%, up to 20%, up to 15%, or any reasonable numeric range constituted by the values noted above as the terminal point, such that the medical implant may exhibit superior effects, e.g., good durability, encapsulation and release profile of the bioactive ingredient, etc. The proper porosity of this layer may be achieved by, e.g., the process of making the medical implant described herein.

In one embodiment, each BACL(s) independently has a thickness of 0.5 µm to 80 µm, e.g., at least 0.1 µm, at least 0.5 µm, at least 1 µm, at least 2 µm, at least 3 µm, at least 4 µm, at least 5 µm, at least 6 µm, at least 7 µm, at least 8 µm, at least 9 µm, at least 10 µm, at least 11 µm, at least 12 µm, at least 13 µm, at least 14 µm, at least 15 µm, at least 20 µm, at least 25 µm, at least 30 µm, at least 35 µm; or at most 80 µm, at most 70 µm, at most 65 µm, at most 60 µm, at most 55 µm, at most 50 µm, at most 45 µm, at most 40 µm; or any reasonable numeric range constituted by the values noted above as the terminal point, e.g., 0.5 µm to 30 µm, 40 µm to 55 µm, 5 µm to 8 µm, etc.

In one embodiment, the adhesion strength between the substrate and BACL is at least or greater than 3B, preferably at least or greater than 4B, more preferably at least 5B, as measured by ASTM D3359.

In one embodiment, each BACL(s) independently has a surface roughness of between 0.05 µm and 4.0 µm, e.g., 0.1 µm to 3.75 µm, 0.2 µm to 3.5 µm, 0.3 µm to 3.25 µm, 0.4 µm to 3 µm, 0.5 µm to 2.5 µm, or any reasonable numeric ranges constituted by any aforementioned terminal point values.

In one embodiment, each BACL(s) independently is formed from primary particles having D50 ranging from 0.1 µm to 10 µm, e.g., being about 0.2 µm, about 0.3 µm, about 0.4 µm, about 0.5 µm, about 0.6 µm, about 0.7 µm, about 0.8 µm, about 0.9 µm, about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 8 µm, about 9 µm, about 10 µm; or any reasonable numeric range constituted by the values noted above as the terminal point, e.g., 0.5 µm to 3 µm, 0.8 µm to 5 µm, etc.

Without being bound to the theory, the BACL is a (nearly) homogenous and continuous layer since it is prepared via AD techniques as disclosed herein. In particular, the BACL may not have apparent single crystals, via the observation of FE-SEM (e.g., 5000X) upon ion milling. In addition, the crystallinity of the BACL, as compared to pure powders of the (composite) materials thereof, may decrease at least 15%, preferably at least 20%, via X-ray diffraction analysis.

### Assisting dense layer (ADL)

In one aspect, the medical implant component further comprises an assisting dense layer (ADL), which is located on the biocompatible substrate (S) and between the biocompatible substrate (S) and the BACL. The ADL is made of a biocompatible metal or alloy, biocompatible ceramic or any mixtures thereof (which may be the same as or different from the material of component (1) of the BACL). The ADL may assist the adhesion between the biocompatible substrate and the BACL.

In one embodiment, the ADL has a thickness of 0.3 µm to 1.5 µm, e.g., at least 0.4 µm, at least 0.5 µm, at least 0.6 µm, at least 0.65 µm, at least 0.7 µm, at least 0.75 µm, at least 0.8 µm, at least 0.85 µm, at least 0.95 µm,; or at most 1.45 µm, at most 1.4 µm, at most 1.35 µm, at most 1.3 µm, at most 1.25 µm, at most 1.2 µm, at most 1.15 µm, at most 1.1 µm, at most 1.05 µm, at most 1 µm; or any reasonable numeric range constituted by the values noted above as the terminal point, e.g., 0.5 µm to 1 µm, 0.4 µm to 1.2 µm, 0.6 µm to 0.95 µm, etc.

In one embodiment, the adhesion strength between the ADL and the biocompatible substrate is at least or greater than 3B, preferably at least or greater than 4B, more preferably at least 5B, as measured by ASTM D3359. In one embodiment, adhesion strength between the the film stack (i.e., the ADL in combination with the BACL) and the biocompatible substrate is at least or greater than 4B, more preferably at least 5B, as measured by ASTM D3359.

In one embodiment, the variation of thickness of the ADL is less than 10%, preferably less than 8%, more preferably less than 5%.

In one embodiment, the ADL has a porosity of less than 1%, preferably of less than 0.8%, more preferably of less than 0.5%.

### Applications of medical implant components

Due to their excellent properties (including, but not limited to, good durability,-good biocompatibility, adjustable drug release rate, etc.), the medical implant components can be used in various applications. Examples of the applications include, but are not limited to, a part or the entirety of an artificial joint (e.g., knee joint replacement, hip joint replacement, shoulder joint replacement, radiocarpal joint replacement; cups, heads, stems, etc.), an insert associated with an artificial j oint, a stent, an intervertebral disc, a screw, an artificial bone plate, an interbody spacer or a permanent or temporary anchorage device (e.g., used in orthodontics, surgery, etc.).

In one embodiment, the surface of the medical implant component has a checkerboard-like pattern. In one embodiment, the surface of the medical implant component has a roughness of 0.3 µm or higher, preferably of 0.8 µm or higher, e.g., of 0.9 µm or higher, of 1 µm or higher, etc. In any preceding embodiments, the variation of thickness of the BACL (the surface structural feature is not included) is less than 10%.

### Process for making medical implant components

Conventional methods for making a coating or layer on the substrate of a medical implant component include plasma spraying, physical vapor deposition (PVD), chemical vapor deposition (CVD), spray processes (thermal or cold spray), sintering processes, etc. The inventors found that using aerosol deposition (AD) to deposit the materials to form the BACL (and optionally the ADL) would be advantageous.

In brief, the AD process may exhibit the following advantages in making the medical implant component described herein: (1) the deposition rate is higher than that for PVD/CVD processes, which has the benefit of increasing the efficiency of production; (2) the temperature for conducting the process for depositing BACL (and optionally ADL) can be significantly lower (e.g., near room temperature) than that for PVD/CVD (e.g., higher than 300°C and even up to 800°C), thermal spray or cold spray (around 300°C) processes; (3) the required degree of vacuum is less restricted than that for conducting PVD/CVD processes; (4) the process may be more easily scaled up compared to PVD/CVD processes; (5) the thickness of the layer can be easily adjusted and may be larger; (6) the adhesion of the deposited layer(s) is significantly higher than that obtained by PVD/CVD, thermal spray or cold spray processes; (7) the density and conformity of the deposited layer(s) may be higher than that obtained by thermal spray or cold spray processes; (8) the deposited layer(s) or coating may be a near-net-shape, which may be difficult when using PVD/CVD, thermal spray or cold spray processes; and (9) the costs may be significant lower than for PVD/CVD, thermal spray or cold spray processes.

As compared to the solution-based techniques, the AD process possesses various advantages over other surface processing techniques. For example, there is no need for pretreatment of the substrate by anodic treatments, as required in other techniques such as immersion with polymer dispersion (optionally further comprising bioactive agents such as antibiotics), solution spray, solution-dropping processes, etc., for forming a porous 3D structure (oxidation, corrosion, etc.). In addition, layers thus-formed by the AD processes may exhibit (minor) surface irregularity, e.g., checkerboard-like pattern, and such (minor) surface irregularity may be beneficial to the desired uses in such as regeneration of cells or tissues of a living body. In one embodiment, the surface irregularity may have an obtuse angle, rather than an acute angle as exhibited in needle-like or sheet-like morphology formed by other surface processing techniques, and may decrease the possibility of repair due to mechanical stress from transportation, packaging, and installation during clinical uses. Without being bound to theory, horizontal (pressed) stripes of the composite materials may be observed in the sectional views, which define spaces (and porosity) to which the bioactive ingredient(s) can be introduced, and thereby distribution of the bioactive ingredient(s) may be more homogeneous than conventional loading processes.

As such, the layer(s) of the medical implant component exhibit high stability and enhanced mechanical strength; the three-dimensional (micro)structure of the layers, which have a porosity, may play an important role in providing mechanical strength, loading and protecting the bioactive ingredient. In addition, there is no need to transfer the semi-products or products during the process of depositing the layers and thus the risk of contaminating the semi-products or products due to transfer (as required by other types of depositing processes) can be eliminated or avoided.

The inventors surprisingly found that AD processes are advantageous in making medical implant components, particularly the BACL.

The present disclosure thus also relates to a process of making a medical implant component described herein, comprising the following steps:
(i) providing a biocompatible substrate (S) in a deposition chamber;
(ii) reducing the pressure in the deposition chamber to less than 2.5 torr, e.g., less than 2.35 torr, less than 2.2 torr; and
(iii) depositing a composite material of a biocompatible-bioactive composite material layer (BACL) via aerosol deposition (AD) to form BACL above or on the biocompatible substrate, and

wherein the composite material comprises component (1): a biocompatible metal or alloy, a biocompatible ceramic or any mixtures thereof, and component (2): an organic bioactive ingredient.

In one aspect, the present disclosure relates to a process of making a medical implant component described herein, comprising the following steps:
(i) providing a biocompatible substrate (S) in a deposition chamber;
(ii) reducing the pressure in the deposition chamber to less than 2.5 torr, e.g., less than 2.35 torr, less than 2.2 torr;
(ii') depositing a material of an assisting dense layer (ADL) on the substrate via aerosol deposition (AD), and
(iii') depositing the composite material of the BACL via aerosol deposition (AD) to form the BACL above or on the ADL, and

wherein the material of the ADL comprises a biocompatible metal or alloy, biocompatible ceramic or any mixtures thereof.

In various embodiments, step (ii') and step (iii') can be conducted at least twice to form multiple biocompatible-bioactive composite material layers (BACLs).

The biocompatible substrate may be those described herein. In one embodiment, the biocompatible substrate is made from a material or materials selected from the group consisting of a biocompatible polymeric material, a biocompatible metal or alloy and a biocompatible ceramic. In one embodiment, the biocompatible polymeric material is selected from the group consisting of polyetheretherketone (PEEK), polyethylene (PE) and polyvinylchloride (PVC). In one embodiment, the PE is low-density polyethylene (LDPE), high-density polyethylene (HDPE) or ultra-high molecular weight polyethylene (UHMWPE).

The AD processes can be conducted via known means and apparatuses. The apparatuses for conducing the AD process may include an aerosol generation unit (comprising, e.g., source(s) of carrier gas(es), a mass flow controller (MFC), an aerosol generating chamber), a deposition chamber (equipped with, e.g., a nozzle/nebulizer, a support plate or stage, etc.) and a vacuum system (e.g., a pump).

Various parameters of the AD processes may be adjusted. Examples of the parameters include, but are not limited to, the type of the carrier gas(es); the flow rate; the distance between the nozzle and the substrate support; the incident angle of the aerosol flow to the substrate; the degree of vacuum in the deposition chamber; the concentration, particle size, types of the material(s) of the a biocompatible protective coating layer in the aerosol flow; the temperature in the chamber; the temperature of the substrate; etc.

Examples of the carrier gas(es) include, but are not limited to, nitrogen (N₂), oxygen (O₂), argon (Ar), helium (He), clean dry air (CDA) and any combinations and proportions thereof. The flow rate of the carrier gas may be 300 to 1500 l/hr, e.g., about 300 l/hr, about 350 l/hr, about 400 l/hr, about 450 l/hr, about 500 l/hr, about 550 l/hr, about 600 l/hr, about 650 l/hr, about 700 l/hr, about 750 l/hr, about 800 l/hr, about 850 l/hr, about 900 l/hr, about 950 l/hr, about 1000 l/hr, about 1100 l/hr, about 1200 l/hr, about 1300 l/hr, about 1400 l/hr, about 1500 l/hr; or any reasonable numeric range constituted by the values noted above as the terminal point, e.g., 500 l/hr to 650 l/hr, 350 to 1300 l/hr, etc.

The temperature (of biocompatible substrate) during the AD process may be controlled in the range from 5 °C to 50 °C, e.g., 15°C to 45°C, preferably no higher than 35 °C.

The material powders may have a D50 ranging from 0.1 µm to 10 µm, e.g., being about 0.2 µm, about 0.3 µm, about 0.4 µm, about 0.5 µm, about 0.6 µm, about 0.7 µm, about 0.8 µm, about 0.9 µm, about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 8 µm, about 9 µm, about 10 µm; or any reasonable numeric range constituted by the values noted above as the terminal point, e.g., 0.5 µm to 3 µm, 0.8 µm to 5 µm, etc. The raw material may be subject to pre-treatment(s), e.g., milling, sieving, etc., to provide the material(s) for the deposition via AD processes.

Once the deposition of the biocompatible-bioactive composite material layer(s) (BACL) (and the optional assisting dense layer (ADL)) is completed, the product may be subject to post-processing, such as cleaning, shaping, etc.

### EXAMPLES

The following examples are provided to make the present invention more comprehensible to those of ordinary skill in the art to which the present invention pertains, but are not intended to limit the scope of the invention.

### Materials, methodologies and test models

Materials of the biocompatible-bioactive composite material layer include Si₃N₄, Al₂O₃, TiN (4N purity), hydroxylapatites, which can be purchased from Sigma-Aldrich.

Materials can be further treated via UPE- Celanese GUR@1020-E, if necessary.

Porosity of the layer can be measured by Hitachi S-4300 FE-SEM. Thickness and roughness can be measured by KLA-D500. Adhesion can be evaluated by ASTM D3359.

Antibacterial activity can be evaluated by JIS Z 2801. In particular, a specimen or composite material layer (e.g., 5 cm x 5 cm) is cleaned and disinfected, and then inoculated with a test suspension comprising a target microorganism(s). The inoculated specimen/composite material layer is covered with a plastic film and incubated at a selected temperature and time, e.g., at 35°C for 24 hours. After incubation, the specimen/composite material layer is washed for calculation of microorganism concentration.

### Example 1

Silicon nitride (D50 of about 1.8 µm, about 25 ml) and powders of vancomycin (about 2 g) were used as the composite material; they were introduced into a nylon can of a milling machine and homogenously mixed at 60 rpm under ambient condition for 3 hours. The mixed powders were placed into an aerosol generator for subsequent procedures.

A Ti substrate was washed by acetone, alcohol and deionized water in an ultrasonic cleaner (each for 10 minutes), dried and then placed into a chamber of the aerosol deposition (AD) device; the chamber was vacummized to 2.2 torr or below. Then, the processing gas (e.g., He) was introduced at a proper flow rate (e.g., 10 to 15 liter/min) to the aerosol generator to produce homogenous aerosols of the mixed powders, which were introduced into the chamber and homogenously sprayed on the substrate to form the BACL. After the AD process was completed, the deposited substrate was retreived and cleaned by clean dry air (CDA) to remove residual powders on the surface thereof, and placed in a dry cabinet for storage and subsequent tests.

### Example 2

Hydroxylapatites (HA) (D50 of about 2.1 µm, about 25ml) and powders of vancomycin (about 2 g) were used as the composite material; they introduced into a nylon can of a milling machine and homogenously mixed at 60 rpm under ambient condition for 3 hours. The mixed powders were placed into a dry carbinet for storage and subsequent procedures.

Pure HA powders were introduced into the container of the aerosol generator. Then, a Ti substrate was washed by acetone, alcohol and deionized water in an ultrasonic cleaner (each for 10 minutes), dried and then placed into a chamber of the aerosol deposition (AD) device; the chamber was vacummized to 2.2 torr or below. The processing gas (e.g., He) was then introduced at a proper flow rate (e.g., 10 to 15 liter/min) to the aerosol generator to produce homogenous aerosols of the HA powders, which were introduced into the chamber and homogenously sprayed on the substrate to form the ADL. After the deposition of ADL was completed, the deposited substrate was retrieved and cleaned by clean dry air (CDA) to remove residual HA powders on the surface thereof. The substrate was again placed into a chamber of the aerosol deposition (AD) device; the chamber was vacummized to 2.2 torr or below. The HA powders were removed from the container of the aerosol generator and the mixed powders of HA and antiboiotics were introduced said container. The processing gas (e.g., He) was then introduced at a proper flow rate (e.g., 10 to 15 liter/min) to the aerosol generator to produce homogenous aerosols of the mixed powders of HA and antibiotics, which were introduced into the chamber and homogenously sprayed on the substrate to form the BACL (on the ADL). After the deposition of BACL was completed, the deposited substrate was retrieved and cleaned by clean dry air (CDA) to remove residual powders on the surface thereof, and placed in a dry cabinet for storage and subsequent tests.

### Example 3 (comparative)

A sample of implant having a surface coating of polymeric material immersed with antibiotics was provided as a comparative example herein. A Ti-6Al-4V substrate was polished subsequently by sandpapers of grit number 400, 600, ... to 1,500, and then immersed into a solution of HF:H₂SO₄ (1M:4M) for 5 minutes; the substrate was cleaned with deionized water in an ultrasonic cleaner for 20 minutes to remove surface residual acids.

Then, an anionization treatment was conducted for the cleaned substrate by immersing it in a solution of 0.2 M Ca(H₂PO₂)₂ (Alfa Aesar, Germany) at 300 V, 100 mA/cm² (from a direct current supplier) for 5 minutes. After treatment, the substrate was rinsed with deionized water for 5 minutes to remove residual anionic solution and dried for subsequent use.

The treated substrate was immersed in a dichloromethane solution of 5% PLGA(Poly D, L-lactide-co-glycolide) and 2 g vancomycin, and drawn up from the solution at a rate of 2 cm/min. The thus-obtained sample was dried at ambient temperature for 30 minutes for subsequent tests.

### Example 4

Samples obtained in Examples 1 to 3 were respectively immersed in 50 mL phosphate buffer physiological solutions (1X PBS, Gibco, thermo fisher scientific, Waltham, USA) at 37°C and shaken on a shaker at 30 rpm for 20 minutes. Then, the subsrates were respectively placed in 200 mL fresh phosphate buffer physiological solutions, and 5mL samples were taken every 12 hours for measuring the concentration of the antibiotics. The antibacterial ability was also evaluated.

Characteristics and test results of the medical implant components set forth in Examples 1 to 3 are listed in Table 1:

**Table 1**

| **Characteristics** | **Ex. 1** | **Ex. 2** | **Ex. 3 (comparative)** |
|---|---|---|---|
| porosity of BACL | 13% | 17% | - |
| thickness of BACL | 11um | 14um | 23um |
| surface roughness | 0.9um | 1.02um | 1.7 |
| adhesion to substrate | 5B | 5B | 3B |
| drug release time (days) | >10 | >10 | 5-6 |
| staphylococcus aureus concentration after 24hr (log CFU/ml) | < 1 | < 1 | > 5 × 10² |
| time for preparing samples | ~ 5hr | ~ 6hr | ~ 12hr |

The maximum drug release time of the invention would be expected to be up to 15 to 23 days or even more. The drug release amount in the first 12 hrs would be expected to be up to 2 mg (e.g., up to 1.7 mg, or at least 1 to 1.2 mg), in the subsequent 36 hrs (i.e., 12^{th} hr to 48^{th} hr) up to 1.4 mg (e.g., up to 1.2 mg, or at least 0.7 mg), and up to 0.8 mg (e.g., up to 0.7 mg, or at least 0.2 mg) after 48 hrs.

The invention apparently exhibits superior effects over the existing medical implant components. In particular, the film structure of the samples obtained in Examples 1 and 2 remains stable and complete after testing for 10 days. FTIR spectra of the BACL of samples in Examples 1 and 2 after testing for 10 days still exhibit the signals of vancomycin, indicating that vancomycin can still release from the film after 10 days. In contrast, the film layer loaded with antibiotics obtained by conventional polymeric coating and direct immersion processes (Example 3) tend to deteriorate and even fall off from the substrate under simulated human physiological conditions, and nearly no vancomycin signals in the FTIR spectra of the polymeric film can be observed for the samples obtained in the tests after 5 or 6 days. In addition, the samples of Examples 1 and 2 passed the antibacterial ability test but the sample of Comparative Example 3 did not, as shown in Table 1 above.

In addition, the AD process can be conducted with simpler procedures, as compared to conventional polymeric-immersion processes, since the powders of the composite materials can be simply prepared by mixing and no liquid-based cleaning would be required. However, conventional polymeric-immersion processes would need complex procedural steps including multiple transfer of substrates, liquid-based cleaning and drying.

In brief, the disclosure provides processes and medical implant components that possess various advantages over the existing techniques, in particular simpler procedures, prolonged release time of bioactive ingredients from the medical implant components and higher structural stability of the components.

A person of ordinary skill in the art of the subject invention should understand that variations and modifications may be made to the teaching and the disclosure of the subject invention without departing from the spirit and scope of the subject application. Based on the contents above, the subject application intends to cover any variations and modifications thereof with the proviso that the variations or modifications or their equivalents fall within the scope as defined in the appended claims.

## Claims

1. A medical implant component, comprising:
(a) a biocompatible substrate (S), and
(b) at least one biocompatible-bioactive composite material layer (BACL), having a porosity of 0.5% to 40%, above or on the substrate;
wherein the biocompatible substrate (S) is made from a material or materials selected from the group consisting of a biocompatible polymeric material, a first biocompatible metal or alloy and a first biocompatible ceramic, and
wherein the BACL is made of a composite material comprising component (1): a second biocompatible metal or alloy, a second biocompatible ceramic or any mixtures thereof, and component (2): an organic bioactive ingredient;
preferably, wherein component (1) in the BACL is selected from the group consisting of oxides of one or more of aluminum, silicon, titanium and zirconium; nitrides of one or more of aluminum, silicon, titanium and zirconium; carbides of one or more of aluminum, silicon, titanium and zirconium; oxide-nitride of one or more of aluminum, silicon, titanium and zirconium; oxide-carbide of one or more of aluminum, silicon, titanium and zirconium; nitride-carbide one or more of aluminum, silicon, titanium and zirconium; oxide-nitride-carbide of one or more of aluminum, silicon, titanium and zirconium; phosphates of calcium; hydroxylapatites; halogenated hydroxylapatites; carbonated hydroxylapatites; halogenated-carbonated hydroxylapatites; and any mixtures of the foregoing.

2. The medical implant component according to Claim 1, wherein the BACL has a thickness of 0.1 µm to 80 µm, 0.5 µm to 50 µm, or 1 µm to 6 µm.

3. The medical implant component according to any preceding claims, wherein the adhesion strength between the substrate and BACL is greater than 3B, as measured by ASTM D3359.

4. The medical implant component according to any preceding claims, which has a surface roughness of 0.3 µm or higher.

5. The medical implant component according to any preceding claims, wherein component (2) in the BACL is selected from the group consisting of antibiotics, platelet-rich plasma (PRP), collagen, steroids, DNA, RNA, antibodies, functional fragments of antibodies; and any mixtures of the foregoing;
preferably, wherein component (2) in the BACL is present in an amount of 0.3 wt.% to 25 wt.%, based on the total weight of the composite material.

6. The medical implant component according to Claim 1, which further comprises an assisting dense layer (ADL), wherein:
the ADL is located between the biocompatible substrate and the BACL,
the ADL is made of a third biocompatible metal or alloy, biocompatible ceramic or any mixtures thereof,
the third biocompatible metal or alloy, biocompatible ceramic or any mixtures thereof are selected from those as defined in Claim 2, preferably different from component (1) of the composite material of the BACL, and
the ADL has (1) a surface roughness of less than 0.3 µm, (2) a porosity of less than 1%, or (3) both (1) and (2).

7. The medical implant component according to Claim 6, wherein the ADL has a thickness of 0.5 µm to 10 µm, preferably the variation of thickness of the ADL is less than 10%.

8. The medical implant component according to Claim 6 or 7, wherein (i) the adhesion between the ADL and the substrate is at least 4B, as measured by ASTM D3359, (ii) the adhesion between the ADL and the BACL is at least 4B, as measured by ASTM D3359 or (iii) both (i) and (ii).

9. The medical implant component according to any preceding claims, which is a part or the entirety of an artificial joint, an insert associated with an artificial joint, a stent, an intervertebral disc, a screw, an artificial bone plate, an interbody spacer or a permanent or temporary anchorage device.

10. The medical implant component according to any preceding claims, wherein the substrate has a surface roughness of at least 0.2 µm.

11. A process of making a medical implant component according to any preceding claims, comprising the following steps:
(i) providing a biocompatible substrate (S), preferably having a surface roughness of at least 0.2 µm, in a deposition chamber;
(ii) reducing the pressure in the deposition chamber to less than 2.5 torr; and
(iii) depositing a composite material of biocompatible-bioactive composite material layer (BACL) via aerosol deposition (AD) to form BACL above or on the biocompatible substrate, preferably the temperature of the substrate ranges from 5°C to 50°C, and
optionally, a step (ii') following the step (ii), and the step (iii) being replaced with a step (iii'):
(ii') depositing a material of an assisting dense layer (ADL) on the substrate via aerosol deposition (AD), wherein the material of the ADL comprises a biocompatible metal or alloy, biocompatible ceramic or any mixtures thereof, preferably different from component (1) of the composite material of the BACL, and
(iii') depositing the composite material of the BACL via aerosol deposition (AD) to form the BACL above or on the ADL;
wherein the composite material comprises component (1): a biocompatible metal or alloy, a biocompatible ceramic or any mixtures thereof, and component (2): an organic bioactive ingredient.

12. The process according to Claim 11, wherein the AD is carried out (1) with a carrier gas selected from the group consisting of N₂, O₂, Ar, He, clean dry air (CDA) and any combinations thereof, (2) at a flow rate of a carrier gas of 300 to 1500 l/hr, and (3) both (1) and (2).

13. The process according to Claim 11 or 12, wherein primary particles of powder of the composite material of the BACL, powder of the material of the ADL or both have a D50 ranging from 0.1 µm to 10 µm.

14. The process according to any of Claims 11 to 13, wherein the biocompatible substrate (S) is made from a material or materials selected from the group consisting of a biocompatible polymeric material, a biocompatible metal or alloy.

15. The process according to any of Claims 11 to 14, wherein step (iii) or step (iii') is conducted at least twice to form multiple BACLs.
